# EUROPEAN PATENT APPLICATION

(11) **EP 0 704 226 A1**
(43) Date of publication of application: **03.04.1996**
(21) Application number: 95115428.5
(22) Date of filing: 29.09.1995
(51) Int. Cl.: A61M 29/02

(54) **Catheter**

(30) Priority: 29.09.1994 JP 234614/94
(71) Applicant: TERUMO KABUSHIKI KAISHA, Tokyo (JP)
(72) Inventor: Ishihara, Kazuhito, c/o Terumo Kabushiki Kaisha, Fujinomiya-shi, Shizuoka (JP); Shibata, Ryuji, c/o Terumo Kabushiki Kaisha, Tokyo (JP)
(74) Representative: Casalonga, Axel

(57) **Abstract**

A guiding catheter for angioplasty or angiography is provided. The guiding catheter has a main lumen near its distal end, and an subsidiary lumen for inflating/deflating the balloon. When the balloon is inflated by introducing a fluid into the subsidiary lumen, the balloon is unfolded to obstruct the main lumen. When therapeutic balloon catheter that has been inserted through the guiding catheter over the guidewire is replaced with a new catheter having a balloon of larger diameter, guidewire may be secured in place by the inflated balloon of the guiding catheter that presses the guidewire against the main lumen wall. Therefore, use of a replacement wire or an extension wire that had been necessary in conventional guiding catheter can be omitted to enable simple and reliable manipulation to relieve the burden of the patient.

## Description

### BACKGROUND OF THE INVENTION

This invention relates to a catheter used in the treatment of coronary vessel, and more particularly, to a catheter having a securing means for enabling an accurate positioning of a catheter of smaller diameter or a guidewire that has been inserted through said catheter.

Noninvasive treatments such as angioplasty and angiography have become popular in the treatment of coronary vessel, and several types of catheters are selectively employed depending on the case and purpose of the treatment.

For example, in percutaneous transluminal coronary angioplasty (PTCA), a balloon catheter having a balloon on its distal end is advanced to the narrowed lesion of the coronary artery of the patient and the catheter is then manipulated to dilate the narrowed lumen of the artery. In such manipulation, a balloon catheter having a diameter corresponding to the narrowed lumen is first introduced into the artery, and the narrowed lesion is dilated to a certain extent by inflating the balloon followed by the withdrawal of the catheter, and subsequently, a new balloon catheter having a larger balloon is introduced to further dilate the lesion. Such procedure is repeated until the coronary artery is dilated to the predetermined diameter.

In such manipulation, replacement of the catheter is carried out as described below.

First, a guiding catheter having a main lumen through which a standard guidewire and a therapeutic balloon catheters may be inserted is introduced to the entrance of the coronary artery where it is maintained, and the standard guidewire is then inserted into the main lumen of the guiding catheter and advanced to the narrowed lesion to be treated.

Next, the therapeutic balloon catheters is inserted into the guiding catheter and advanced over the standard guidewire to the stenotic lesion to treat the lesion with the therapeutic balloon catheter. After the treatment, the therapeutic balloon catheters is withdrawn from the guiding catheter for replacement with the next therapeutic balloon catheters having a balloon of larger diameter. To maintain the guidewire in the predetermined place, it is necessary to secure the proximal end of the guidewire to thereby avoid the guidewire to be simultaneously pulled out of the vessel with the therapeutic catheter.

If the position of the guidewire is not maintained and the distal end of the guidewire is displaced off the predetermined place, reinsertion of the guidewire would be necessary requiring extra time and effort as well as additional X-ray irradiation.

Length of the proximal end of the standard guidewire is generally shorter than the length of the therapeutic balloon catheter to be removed, and change of the standard guidewire to a replacement guidewire of longer length (full length of about 300 cm) is thus required before the withdrawal of the therapeutic balloon catheter. Such manipulation is quite complicated since the guidewire is frequently displaced in such procedure and handling of the extra replacement guidewire is troublesome.

Methods for changing the therapeutic catheter without using any replacement guidewire are disclosed in Japanese Patent Application Laid-Open Nos. 62 (1987)-68465, 2(1990)-4390 and 2(1990)-31765 wherein the standard guidewire is provided with a joint at its proximal end, to which an extension wire may be connected before changing the therapeutic catheter so that the thus elongated guidewire would have a length comparable to the replacement guidewire.

At a glance, such system might seem convenient to allow change of the therapeutic catheter by a simple procedure. Such system, however, requires improvements of the defects as described below.

In the first place, connection of the extension guidewire to the standard guidewire is associated with high risk of displacement of the standard guidewire by the force applied in axial direction upon the connection of the extension guidewire, and confirmation of the guidewire position by X ray irradiation would then be necessary after each connection procedure. In addition, connection of the extension guidewire to the standard guidewire in accordance with the prior art was irreversible, and disconnection of the extension guidewire required cutting of the wire at the joint portion.

In the second place, connection of the extension guidewire to the standard guidewire should leave a substantial bump or step at the joint, which may interfere with the withdrawal of the therapeutic catheter.

Another method for removing and replacing a coronary balloon catheter during coronary angioplasty is disclosed in USP 5,395,389, wherein the guidewire is secured by another balloon catheter inserted in the guiding catheter. In this method, (i) an extra balloon catheter should be inserted into the guiding catheter in addition to the therapeutic catheter, and therefore, a considerable skill would be required for the manipulation; (ii) insertion of the extra balloon catheter may result in the displacement of the guidewire; and (iii) the guidewire is not reliably secured since the balloon of the extra balloon catheter is not fixedly secured to the guiding catheter. Accordingly, this system still needs improvements.

### SUMMARY OF THE INVENTION

In view of the above-described situation, an object of the present invention is to provide a catheter system for treatment of coronary vessel such as coronary angioplasty, angiography, or the like utilizing a plurality of therapeutic catheters, that would enable a smooth change of the therapeutic catheter with no use of replacement guidewire, extension guidewire, or the like.

In order to achieve such object, there is provided by the present invention a catheter having a distal end and a proximal end provided with a main lumen axially extending between the distal end and the proximal end through which a therapeutic tubular member and/or a guidewire or a liquid is to be introduced; wherein said catheter has at least one subsidiary lumen; and wherein an inflatable balloon is provided in the catheter in its distal portion such that the balloon obstructs the main lumen upon its inflation; said balloon being inflated and/or deflated by a gas or a liquid introduced and/or removed through at least one of said subsidiary lumen which is in communication with the balloon. In the catheter of the present invention, the balloon is provided to leave no substantial step in tangential direction at the joint between the balloon and the main lumen wall when the balloon is deflated, and the balloon comprises a substantially gas non-permeable material.

### BRIEF DESCRIPTION OF THE DRAWINGS

FIG. 1 is a schematic view of a guiding catheter according to the present invention.

FIG. 2(A) is a transverse cross-section of the guiding catheter of FIG. 2(C) taken along lines A-A.

FIG. 2(B) is a transverse cross-section of the guiding catheter of FIG. 2(C) taken along lines B-B when the balloon is defrated.

FIG. 2(C) is a partial longitudinal cross-section of an embodiment of the guiding catheter according to the present invention.

FIG. 3(A) is a transverse cross-section of the guiding catheter of FIG. 3(C) taken along lines A-A.

FIG. 3(B) is a transverse cross-section of the guiding catheter of FIG. 3(C) taken along lines B-B when the balloon is defrated.

FIG. 3(C) is a partial longitudinal cross-section of another embodiment of the guiding catheter according to the present invention.

### DESCRIPTION OF THE INVENTION

The present invention is described in further detail.

The procedure for removing and replacing the therapeutic balloon catheter by using the guiding catheter of the present invention comprises the steps of:
(1) withdrawing the therapeutic catheter to a position proximal to the balloon provided in the distal portion of the guiding catheter with the proximal end of the guidewire held in place;
(2) inflating the balloon by introducing a liquid or a gas into the subsidiary lumen provided in the side wall of the guiding catheter to thereby press the guidewire against the side wall of the main lumen by the inflated balloon to prevent the movement of the guidewire (In the meanwhile, the tip of the guidewire stays in place at the predetermined position in the vessel.);
(3) removing the therapeutic catheter, and inserting the next therapeutic catheter;
(4) advancing the new therapeutic catheter to a position proximal to the inflated balloon in the main lumen, and confirming that the proximal end of the guidewire extends out of the therapeutic catheter hub; and
(5) deflating the balloon of the guiding catheter and advancing the new therapeutic balloon catheter over the guidewire until it reaches the stenotic lesion with the proximal end of the guidewire held in place.

As described above, use of the catheter according to the present invention would enable the therapeutic catheter to be changed in a reliable and simple manner without using any replacement guidewire or extension guidewire. As a consequence, operation period and burden on the blood vessel would be reduced, relieving the load of the patient. Such simplified operation should also save the labor of the physician and greatly contribute for the prevention of careless mistakes.

The balloon of the present catheter may comprise a membrane of high gas barrier properties so that the inflation pressure of the balloon may be maintained for a sufficient period of time when the balloon is inflated by means of a gas. Preferable materials include polyethylene, polyethylene terephthalate, polyamide, modified polyolefin, EVA, PVC, and the like.

The balloon of the present catheter may alternatively comprise a highly inflatable material such as a latex, a silicone rubber, or a plastic elastomer. In such case, volume of the guiding catheter main lumen obstructed by the balloon may be freely adjusted by deforming the balloon material through inflation, and size of the uninflated balloon may be reduced.

The balloon may preferably have a diameter equal to the inner diameter of the guiding catheter, and when the balloon comprises a highly compliant material, the guidewire may be secured to the main lumen wall with a larger force by increasing the inflation pressure of the balloon depending on the desired extent of the securing of the guidewire.

The force by which the guidewire is pressed against the main lumen wall may increase with the increase in contact area of the inflated balloon and the side wall of the main lumen of the guiding catheter. However, when the contact area excessively extends in axial direction, namely, when the length of the uninflated balloon over which the therapeutic catheter should be withdrawn before the securing of the guidewire by the balloon is excessively long, use of a longer guidewire would be necessary. In view of such balance, the balloon may preferably have an axial length of from 2 to 80 mm, and more preferably, a length of from 2 to 40 mm.

The length of the balloon is also limited by operative and functional convenience to no longer than 100 mm since an excessively long guidewire should arise various operative and functional difficulties.

The balloon can be provided at any axial position within the guiding catheter, and the position is primarily limited by the length of the guidewire employed. A guidewire of 180 cm in length is generally used for angioplasty, and in such a case, the balloon for securing the guidewire during the replacement of the therapeutic balloon catheter may preferably be provided at a position 5 to 40 cm and more preferably 10 to 20 cm from to the distal end of the guiding catheter. It should be noted that provision of the balloon within the shaped portion at the distal end of the guiding catheter should be avoided. When a longer guidewire having a length exceeding 180 cm is employed, it would then be possible to provide the balloon at a position nearer to the proximal end of the guiding catheter. For example, when a guidewire of 220 cm is employed, the balloon can provided near the hub of the guiding catheter.

The guiding catheter of the present invention may be so constituted that its flexibility would increase from the proximal end to the distal end. To prevent alteration in diameter of the guiding catheter upon inflation of the balloon, the distal end portion of the guiding catheter should have a Shore hardness in the range of from 40A to 70D, and preferably, from 70A to 65D. The materials that meet such requirement include polyurethanes, polyamides, polyethylene terephthalates, fluoroplastics, fluoroplastic elastomers, and polymer alloys thereof. The guiding catheter may comprise a monolayer or a multilayer structure of such material.

It is also preferable to provide a reinforcement coil or mesh comprising a stainless steel, a Ni-Ti alloy, tungsten, or an amorphous alloy with the guiding catheter so that, when inserted in the blood vessel, the force applied to the proximal end of the guiding catheter in axial and/or tangential direction may be effectively transmitted along full length of the catheter to its distal end, that is, for the purpose of improving torque transmission of the guiding catheter. Such reinforcement coil or mesh may be provided from proximal end to the intermediate portion of the guiding catheter.

The guiding catheter of the present invention may preferably have a marker of an X-ray contrast medium on its distal end portion so that the physician can readily confirm the position of the balloon during the operation. The X-ray contrast medium may be selected from a synthetic resin having kneaded therein a powder of barium sulfate, zirconium oxide, bismuth oxide, bismuth bicarbonate, etc., or a metal such as gold, platinum, silver, or tungsten. It would be preferable to provide one or more marker on exterior of the guiding catheter and/or on the periphery of the main lumen on the circumference corresponding to at least one axial end of the balloon

The main lumen of the guiding catheter may have different diameter depending on the purpose and usage of the guiding catheter, and a diameter of from 1.3 to 3.0 mm is generally preferred.

The guidewire and the therapeutic catheter are respectively inserted from the exterior of the blood vessel to the treatment site via the main lumen of the guiding catheter, and during the passage through the main lumen, they may get caught over the bump or the step in the main lumen, which may result in the undesirable displacement of the guidewire or scratch on the balloon surface. Therefore, no substantial step or bump should be left on the side wall of the main lumen at the position of the balloon.

The balloon and the subsidiary lumen may be constructed as a single tubular member, and in such a case, the balloon portion of the tubular member should contact the walls of the main lumen at an acute angle so as leave substantially no step at the joint.

When the guiding catheter of the present invention is employed, the guidewire will be secured against the interior of the guiding catheter at a position relatively close to the distal end of the guidewire, that is, at a position closer to the stenotic lesion of the blood vessel, and therefore, a reliable manipulation would be enabled since the guidewire is far less likely to be displaced during the manipulation compared to conventional catheters.

In addition, since the balloon is fixedly secured to the main lumen at least at its proximal and distal ends, the guidewire is less likely to become displaced during the securing procedure and the guidewire will be reliably be secured in place.

The present invention is described in further detail by referring to the Examples of the invention and drawings, which by no means limit the scope of the invention.

### Examples

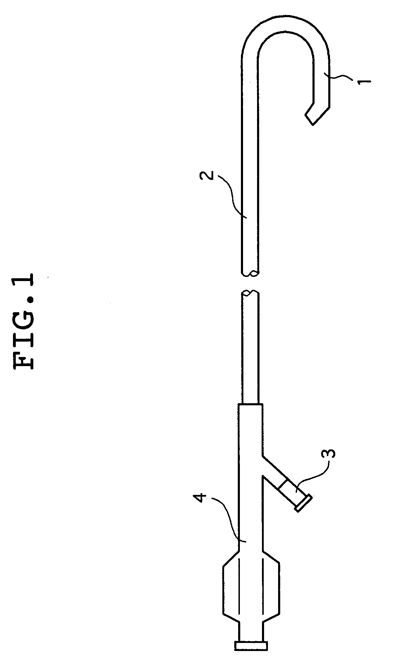

FIG. 1 is a schematic view of the guiding catheter according to the present invention. The guiding catheter of FIG. 1 comprises a main catheter portion 2, and the main catheter portion is provided with a hub 4 on its proximal end, and with a shaped portion 1 on its distal end. The hub 4 is provided with a balloon inflation/deflation coupler 3 to which a balloon inflator/deflator may be connected for inflating/deflating the balloon.

When the balloon is fabricated form an elastomeric material such as a rubber such as latex or a plastic elastomer, the coupler 3 may be provided with a stop cock, which may be opened with a syringe to inflate the balloon, closed for maintaining the inflated state of the balloon, and re-opened to allow for automatic deflation by resilience of the balloon material.

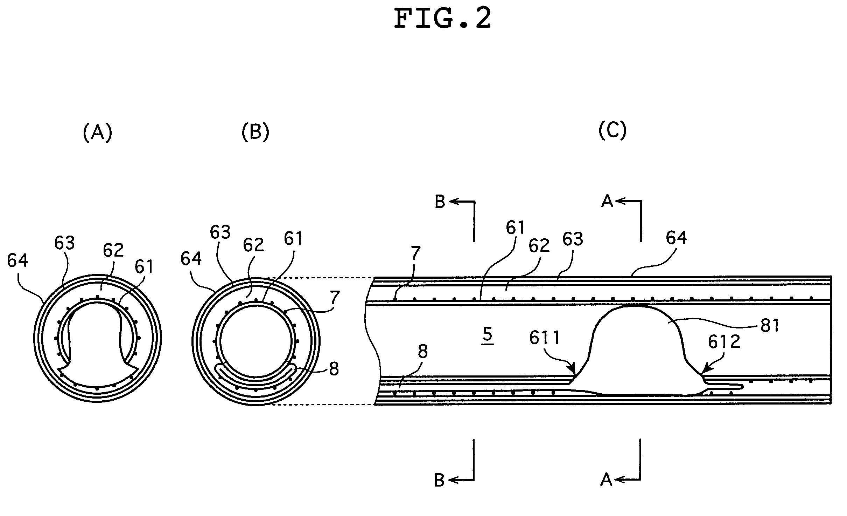

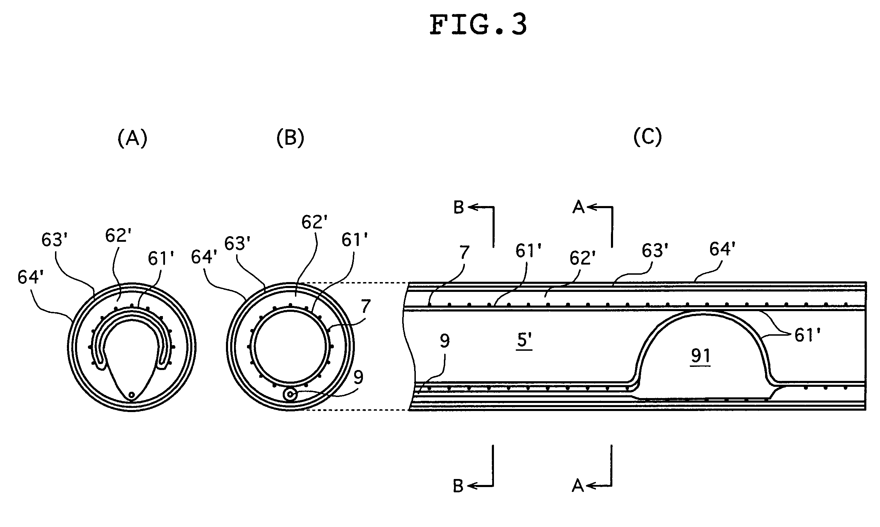

FIGS. 2 and 3 are partial cross-sectional views of typical embodiments of the guiding catheters according to the present invention.

FIG. 2 is a cross-sectional view of the part of the guiding catheter according to an embodiment of the present invention that includes the balloon.

The guiding catheter of this embodiment has a main lumen 5 axially extending through the catheter from its proximal end to the distal end. A balloon 81 is fixedly secured in the distal portion near the distal end, and when inflated, the balloon 81 completely obstructs the main lumen 5 of the guiding catheter.

The balloon 81 of this guiding catheter comprises a thermoplastic resin which may be selected from those commonly used for balloons used in PTCA.

The balloon 81 has a diameter after inflation of about 2.25 mm while the catheter has an inner diameter of 2.1 mm and an outer diameter of 2.37 mm.

The balloon 81 is inflated by a fluid introduced through a subsidiary lumen 8 provided for balloon inflation. The subsidiary lumen 8 in communication with the balloon 81 is provided within the wall of the guiding catheter between the exterior surface of the guiding catheter and the main lumen 5. The proximal end of the subsidiary lumen (lumen tube) 8 terminates at the balloon inflation/deflation coupler 3 and the distal end of the subsidiary lumen 8 terminates at the balloon 81.

It is preferable to preliminarily fabricate a lumen tube 8 connected to the uninflated balloon 81 so that the interior of the lumen tube 8 would be in fluid communication with the interior of the balloon 81. The lumen tube 8 that has been connected to the balloon 81 may then be axially laid on a tubular member that constitutes the main lumen 5 of the guiding catheter and that has an opening at the position over which the balloon 81 is to be laid. An outer layer of the guiding catheter may then be formed on the main lumen tube 5 to embed at least the lumen tube 8 and proximal and distal ends of the balloon 81.

In the case of the embodiment shown in FIG. 2, the catheter wall comprises four tubular layers, namely, first layer 61, second layer 62, third layer 63 and fourth layer 64. The first layer 61 constitutes the main lumen 5, and has an opening at the position corresponding to the balloon 81. The proximal and distal ends of the opening are designated 611 and 612. The first layer 61 was formed of PTFE resin having 0.02 to 0.080 mm thick, the second layer 62 was formed of a polyamide, a polyester, a polyurethane or an erastomer thereof having 0.030 to 0.110 mm thick, the third layer 63 was formed of a polyamide, a polyester, a polyurethane or an erastomer thereof having 0.05 to 0.100 mm thick, the fourth layer 64 was formed of a polyamide, a polyester, a polyurethane or an erastomer thereof having 0.030 to 0.070 mm thick and metal wire 7 was formed of SUS or Ni-Ti alloy, further, the subsidiary lumen 8 and the balloon 81 was formed of PET, PPS (polyphenylene sulfide), a silicone, a polyamide, a polyester, a polyurethane or an erastomer thereof having 0.21 to 0.016 mm thick. In the production of the guiding catheter of this embodiment, the lumen tube that constitutes the subsidiary lumen 8 and the uninflated balloon 81 are laid along the first layer 61 constituting the wall of the main lumen 5, and a metal wire 7 is wound thereover, and the overlying second layer is then formed by dipping or extrusion molding to embed the lumen tube 8 and the proximal and the distal ends of the balloon 81. Third and forth layers 63 and 64 may then be formed over the thus formed second layer 62. As a consequence, the balloon 81 of the guiding catheter of this embodiment is accommodated in folded state in the recess formed in the interior of the main lumen 5 whose proximal and distal ends are designated 611 and 612 until its inflation and unfolding.

It should be noted that the metal wire 7 may be wound on the first layer except for the area of the opening before the laying of the lumen tube 8 and the uninflated balloon 81.

Alternatively, the first layer 61 constituting the main lumen 5 may be provided with two openings 611' and 622', and the lumen tube 8 that has been preliminarily connected to a balloon 81 may be laid on the first layer 61 with the balloon 81 entering the main lumen 5 from the opening 611' with its the distal end exiting the main lumen 5 at the opening 612'. The second layer is then formed on the first layer 61 to embed the lumen tube 8 and the proximal and distal end portions of the balloon 81.

FIG. 3 shows another embodiment of the guiding catheter according to the present invention in partial cross-sectional view.

The guiding catheter of this embodiment also has a main lumen 5' axially extending through the catheter from its proximal end to the distal end and comprises four layers, namely, first layer 61', second layer 62', third layer 63' and fourth layer 64'. The first layer 61' may preferably comprise a highly resilient material such as a silicone rubber, a latex rubber, or a plastic elastomer. The first layer 61', the second layer 62', the third layer 63', the fourth layer 64' and metal wire 7' were formed of the same resin materials having the same sizes, further, the subsidiary lumen 9 and the balloon 91 were similarly formed as described in Fig. 2. The balloon 91 and the subsidiary lumen 9 for inflating the balloon 91 that is in communication with the balloon 91 are embedded in the second layer 62' disposed on the first layer 61'. As in the case of the above-described embodiment, the guiding catheter of this invention is fabricated by laying down the lumen tube 9 and the balloon 91 and the first layer 61' constituting the main lumen 5' within the wound metal wire 7'; and forming the second layer 62' on the exterior of the wire 7' by dipping or extrusion molding. The metal wire 7' may be alternatively disposed on the first layer 61' except for the area corresponding to the balloon 91. When the balloon 91 is inflated, the corresponding portion of the first layer 61' comprising a highly resilient material would simultaneously expanded with the balloon 91.

As described above, the guiding catheter of the present invention is provided with a balloon near its distal end, and a subsidiary lumen for inflating the balloon. Therefore, when replacement of a therapeutic balloon catheter is required, the therapeutic balloon catheter may be withdrawn to a position proximal to the balloon of the guiding catheter with the guidewire being maintained in position by holding it at its proximal end, and the balloon can be subsequently inflated to press the guidewire against the wall constructing the main lumen. Once the guidewire is reliably secured, the therapeutic catheter to be removed may be withdrawn from the guiding catheter and a replacement therapeutic catheter may be inserted into the guiding catheter and advanced over the guidewire. When the proximal end of the guidewire is exposed, the guidewire is maintained in position by holding it at its proximal end, and the balloon is deflated to free the guidewire at the position of the balloon. The therapeutic catheter is then advanced over the guidewire to the stenotic lesion.

The guiding catheter of the present invention would enable change of the therapeutic catheter by simplified procedure with no use of the replacement guidewire or extension guidewire to realize a faster manipulation with a higher reliability. Accordingly, the burden of the patient could be markedly relieved by the use of the catheter according to the present invention.

In addition, since the guidewire is secured at a position in the vicinity of its distal end, namely, in the vicinity of the stenotic lesion of the blood vessel by the balloon whose proximal and distal ends are fixedly secured to the guiding catheter, the guidewire is less likely to become displaced during the manipulation to enable a far more reliable manipulation.

Moreover, since the subsidiary lumen is embedded in the wall of the guiding catheter and the deflated balloon is tightly accommodated on the wall of the main lumen, smooth advancement and withdrawal of the guidewire as well as the therapeutic catheter is realized.

## Claims

1. A catheter having a distal end and a proximal end provided with a main lumen axially extending between the distal end and the proximal end through which a therapeutic tubular member and/or a guidewire or a liquid is to be introduced;
wherein said catheter has at least one subsidiary lumen; and
wherein an inflatable balloon is provided in the catheter in its distal portion such that the balloon obstructs the main lumen upon its inflation; said balloon being inflated and/or deflated by a gas or a liquid introduced and/or removed through at least one subsidiary lumen which is in communication with the balloon.

2. The catheter according to claim 1 wherein said balloon is smoothly provided to leave no substantial step in tangential direction between the balloon and the wall constructing the main lumen when said balloon is deflated.

3. The catheter according to claim 1 wherein said balloon comprises a gas barrier material.

4. The catheter according to claim 1 wherein said catheter has a marker comprising an X-ray contrast medium in the vicinity of its distal end.
